# EUROPEAN PATENT APPLICATION

(11) **EP 3 838 265 A1**
(43) Date of publication of application: **23.06.2021**
(21) Application number: 19850475.5
(22) Date of filing: 14.08.2019
(51) Int. Cl.: A61K 9/107, A61K 31/4545, A61P 7/04, B82Y 5/00, B82Y 40/00

(54) **INJECTABLE PHARMACEUTICAL COMPOSITION AND PREPARATION METHOD THEREFOR**

(30) Priority: 14.08.2018 CN 201810919343
(71) Applicant: Jiangsu Hengrui Medicine Co., Ltd., Lianyungang, Jiangsu 222047 (CN)
(72) Inventor: LIU, Ting, Lianyungang, Jiangsu 222047 (CN); SUN, Qiong, Lianyungang, Jiangsu 222047 (CN); SHI, Congjian, Lianyungang, Jiangsu 222047 (CN); CHEN, Xinxin, Lianyungang, Jiangsu 222047 (CN); ZHANG, Yating, Lianyungang, Jiangsu 222047 (CN); LIU, Kai, Lianyungang, Jiangsu 222047 (CN)
(74) Representative: dompatent von Kreisler Selting Werner - Partnerschaft von Patent- und Rechtsanwälten mbB
(86) International application number: PCT/CN2019/100558
(87) International publication number: WO 2020/034989

(57) **Abstract**

An injectable pharmaceutical composition and a preparation method therefor. Specifically, the pharmaceutical composition comprises apixaban nanoparticles, a surface stabilizer, and may further comprise a sedimentation inhibitor. The pharmaceutical composition has good stability and is suitable for industrial mass production.

## Description

### Cross reference to related application

The present application claims priority of the Chinese Patent Application CN201810919343.6 filed on Aug 14, 2018. The contents of which is incorporated herein by reference in its entirety.

### Technical Field

The present invention relates to the fields of pharmaceutical formulations. Specifically, it relates to an injectable pharmaceutical composition comprising apixaban nanoparticles and a preparation method therefor.

### Backgroud

Apixaban is a white to light yellow powder, which is not ionized at physiological pH (1.2 to 6.8) and its solubility in the physiological pH range is about 0.04 mg/ml. The LogP of Apixaban is 3.53, which has good permeability. Apixaban is clinically used for the prevention and treatment of thrombosis, especially for the prevention of venous thromboembolic events (VTE) in adult patients undergoing elective hip or knee replacement. The oral bioavailability of apixaban is not high, approximately 50%, and solubility of which is a limiting factor for its absorption. Apixaban has the advantages of high selectivity, high safety, no need for routine detection of coagulation function, little interaction with food, and no need to adjust dosage according to age, gender, constitution and race of patients. Currently, commercially available apixaban tablets are taken orally twice a day, with single dosage form and poor oral bioavailability. In addition, tablets are not easy to swallow for children and comatose patients, so it is of great clinical value to develop new dosage forms of apixaban that are safe, effective and convenient.

WO2011106478 discloses a pharmaceutical composition comprising crystalline apixaban particles having a D90 equal to or less than about 89 µm and a pharmaceutically acceptable sedimentation inhibitor or carrier. CN104736142A discloses an oral liquid formulation comprising apixaban, water, and at least two solubilizers. CN101340933A discloses an injectable formulation comprising substituted β-cyclodextrin and apixaban. CN102802608A discloses a solubility-improved form of apixaban, which provides controlled release of apixaban.

### Content of the present invention

The present invention provides an injectable pharmaceutical composition comprising apixaban nanoparticles and a surface stabilizer.

The present invention also provides a stable injectable pharmaceutical composition comprising apixaban nanoparticles and a surface stabilizer.

The nanoparticles may be prepared by co-milling, high-pressure homogenization, or anti-solvent method, preferably co-milling. A liquid medium for co-milling may be selected from one or more of water, brine solution, safflower seed oil, ethanol, tert-butanol, hexane and ethylene glycol, preferably one or more of water, brine solution and safflower seed oil, most preferably water. The preferred solution of the present invention is to use water as the liquid medium and the apixaban is co-milled with the surface stabilizer to prepare nanoparticles.

The average particle size or D50 of the apixaban nanoparticles may be less than 2000 nm, preferably less than 1000 nm, more preferably less than 500 nm, furthermore preferably less than 200 nm, further preferably less than 170 nm, furthermore preferably less than 160 nm, most preferably less than 150 nm.

The surface stabilizer may be a nonionic, an anionic, a cationic or a zwitterionic compound or surfactant.

The surface stabilizer may be one or more of nonionic surface stabilizers, anionic surface stabilizers, cationic surface stabilizers and zwitterionic surface stabilizers.

The surface stabilizer may be selected from one or more of povidone, polyvinyl alcohol, docusate sodium, hydroxypropyl methylcellulose, Tween 80, poloxamer, polyethylene glycol 15-hydroxystearate, lecithin, sodium deoxycholate, sodium cholate, preferably one or more of poloxamer, sodium deoxycholate, povidone, docusate sodium and Tween 80, further preferably sodium deoxycholate and povidone.

The surface stabilizer used in the pharmaceutical compositions of the present invention does not comprise sodium laurylsulfonate or sodium dodecyl sulfate, which is not suitable for injection.

The nonionic surface stabilizers of the present invention can include, but are not limited to, one or more of hydroxypropyl methylcellulose (HPMC), polyvinyl alcohol, povidone, poloxamer, Tween 80, and polyethylene glycol 15-hydroxystearate, preferably one or more of povidone, poloxamer and Tween 80.

The anionic surface stabilizers of the present invention can include, but are not limited to, one or more of dioctyl sodium succinate (DOSS), docusate sodium, sodium cholate and sodium deoxycholate, preferably docusate sodium and/or sodium deoxycholate, further preferably sodium deoxycholate.

The cationic surface stabilizers of the present invention can include, but are not limited to, one or more of poly-N-methylpyridinium, pyridinium chloride sulfate, cationic phospholipid, chitosan, polylysine, polyethylene imidazole, polystyrene, polymethylmethacrylate trimethylammonium bromide (PMMTMABr), hexylmethyl trimethyl ammonium bromide (HDMAB), and polyvinylpyrrolidone-2-dimethylaminoethyl dimethyl methacrylate sulfate.

The zwitterionic surface stabilizers of the present invention can include, but are not limited to, one or more of protein, phospholipid, and zwitterionic surfactant, and may be, for example, one or more of phosphatidylcholine, amino acid surfactant, beet alkaline surfactant, lecithin and gelatin.

The surface stabilizers of the present invention can not only comprise surface stabilizers as described above in the traditional sense, but can also comprise some additives that are equivalent to the above surface stabilizers, for example, the mixture of deoxycholic acid and sodium phosphate is equivalent to sodium deoxycholate.

The weight ratio of the apixaban nanoparticle to the surface stabilizer may be selected from 1:0.01 to 1:100, preferably 1:0.1 to 1:10, more preferably 1:0.2 to 1:5, further preferably 1:0.3 to 1:4 (e.g., 1:0.2, 1:0.3, 1:0.45, 1:1.725 or 1:3.1) or 1:0.3 to 1:3.

The surface stabilizer of the present invention can comprise a first surface stabilizer and/or a second surface stabilizer. When the first surface stabilizer is poloxam, the surface stabilizer further comprises the second surface stabilizer.

The first surface stabilizer may be selected from the nonionic surface stabilizer and/or the zwitterionic surface stabilizer, preferably one or more of povidone, polyvinyl alcohol, hydroxypropyl methylcellulose, Tween 80, poloxamer, polyethylene glycol 15-hydroxystearate and lecithin, more preferably one or more of povidone, poloxamer and Tween 80.

The second surface stabilizer may be selected from the anionic surface stabilizer, preferably one or more of sodium deoxycholate, sodium cholate and docusate sodium, more preferably sodium deoxycholate or docusate sodium, most preferably sodium deoxycholate.

The weight ratio of the apixaban nanoparticle to the first surface stabilizer may be selected from 1:0.01 to 1:10, preferably 1:0.1 to 1:10, more preferably 1:0.1 to 1:5, further preferably 1:0.1 to 1:3, furthermore preferably 1:0.2, 1:0.3, 1:1.15, or 1:2.1.

The weight ratio of the apixaban nanoparticle to the second surface stabilizer may be selected from 1:0.01 to 1:10, preferably 1:0.1 to 1:5, more preferably 1:0.1 to 1:2 or 1:0.1 to 1:1, furthermore preferably 1:0.1, 1: 0.15, 1:0.575 or 1: 1.

The average particle size or D50 of the apixaban nanoparticle may be less than 2000 nm, preferably less than 1000 nm, more preferably less than 500 nm, furthermore preferably less than 200 nm, further preferably less than 170 nm, furthermore preferably less than 160 nm, most preferably less than 150 nm.

A method for preparing the pharmaceutical composition of the present invention, comprising: co-milling the surface stabilizer with the apixaban to prepare the pharmaceutical composition comprising apixaban nanoparticles.

The method for preparing the pharmaceutical composition of the present invention, may further comprise: co-milling the first surface stabilizer and/or the second surface stabilizer with the apixaban to prepare the pharmaceutical composition comprising apixaban nanoparticles.

The present invention provides a technical solution for co-milling the apixaban with a portion of or all the surface stabilizer to obtain the pharmaceutical composition comprising apixaban nanoparticles.

In some embodiments, if the apixaban is co-milled with all the surface stabilizer, which means that the pharmaceutical composition comprises only the surface stabilizer used in co-milling and no further surface stabilizer will be added in other processes, the weight ratio of the apixaban to the surface stabilizer may be selected from 1:0.01 to 1:10, preferably 1:0.1 to 1:10, more preferably 1:0.2 to 1:5 (e.g., 1: 0.2, 1:0.3, 1:0.45, 1:1.725 or 1:3.1), further preferably 1:0.3 to 1:4 or 1:0.3 to 1:1. The surface stabilizer can comprise the first surface stabilizer and/or the second surface stabilizer, the weight ratio of the apixaban to the first surface stabilizer may be selected from 1:0.01 to 1:10, preferably 1:0.1 to 1: 10, more preferably 1:0.1 to 1:5, furthermore preferably 1:0.1 to 1:3, further preferably 1:0.2, 1:0.3, 1:1.15 or 1:2.1; and the weight ratio of the apixaban to the second surface stabilizer may be selected from 1:0.01 to 1:10, preferably 1:0.1 to 1:5, more preferably 1:0.1 to 1:2, further preferably 1:0.1, 1: 0.15, 1:0.575, or 1: 1.

In some embodiments, the apixaban is co-milled with a portion (i.e., the remaining portion) of the surface stabilizer which is mixed into the pharmaceutical composition during dilution. The ratio of the surface stabilizers co-milled with the apixaban to the entire surface stabilizers may be selected from 1:2 to 1:100, preferably 1:2 to 1:50, more preferably 1:2 to 1:20, further preferably 1:3 to 1:15 or 1:3 to 1:10, further preferably 1:5.75 or 1:10.3. According to this embodiment, too much foaming which affecting the process during the milling process due to adding too much surface stabilizer at the beginning may be avoided, and the diluted pharmaceutical composition may be stabilized by adding the surface stabilizer. Two portions of the surface stabilizers may be the same kind of surface stabilizers or different kinds of surface stabilizers, and the preferred solution is that two portions of the surface stabilizers are the same kind. When the apixaban is co-milled with a portion of the surface stabilizer, the weight ratio of the apixaban to the portion of the surface stabilizer may be selected from 1:0.01 to 1:10, preferably 1:0.1 to 1:10, more preferably 1:0.2 to 1:5, most preferably 1:0.3 to 1:1. The portion of the surface stabilizer can comprise the first surface stabilizer and/or the second surface stabilizer, the weight ratio of the apixaban to the first surface stabilizer may be selected from 1:0.01 to 1:10, preferably 1:0.1 to 1:10, more preferably 1:0.1 to 1:5, most preferably 1:0.1 to 1:0.3 (e.g. 1:0.2); the weight ratio of the apixaban to the second surface stabilizer may be selected from 1:0.01 to 1:10, preferably 1:0.1 to 1:5, more preferably 1:0.1 to 1:1, most preferably 1:0.1 to 1:0.5. The other portion (i.e., the remaining portion) of the surface stabilizer is diluted with the pharmaceutical composition comprising apixaban nanoparticles, and the solvent used for dilution may be selected from the liquid medium as described above. The dilution factor may be selected from 1 to 100 times, preferably 5 to 50 times, most preferably 10 to 30 times, without limiting the order of mixing. The weight ratio of the apixaban to the other portion (i.e., the remaining portion) of the surface stabilizer may be selected from 1:0.01 to 1:10, preferably 1:0.1 to 1:10, more preferably 1:0.1 to 1:5, most preferably 1:1 to 1:3. The other portion (i.e., the remaining portion) of the surface stabilizer can comprise the first surface stabilizer and/or the second surface stabilizer, and the weight ratio of the apixaban to the first surface stabilizer may be selected from 1:0.01 to 1:10, preferably 1:0.1 to 1:10, more preferably 1:0.1 to 1:5 (e.g. 1:0.95, 1:1.9), most preferably 1:1 to 1:3; the weight ratio of the apixaban to the second surface stabilizer may be selected from 1:0.01 to 1:10, preferably 1:0.1 to 1:5 (e.g. 1:0.9, 1:2), more preferably 1:0.1 to 1:1, most preferably 1:0.3 to 1:1.

The pharmaceutical composition of the present invention can also comprise a sedimentation inhibitor. The sedimentation inhibitor inhibits the sedimentation or aggregation of nanoparticle for a certain period of time.

The sedimentation inhibitor may be selected from one or more of sugar, polyol, and polymer, such as one or more of mannitol, sucrose, albumin, dextran 40, trehalose, glycerol, povidone, glycine, glycerol and hydroxypropyl-β-cyclodextrin, preferably one or more of mannitol, sucrose, and dextrose 40, further preferably mannitol or sucrose, more preferably mannitol. The weight ratio of the apixaban nanoparticle to the sedimentation inhibitor may be selected from 1:0.1 to 1:100, preferably 1:0.1 to 1:50, more preferably 1:0.5 to 1:30 (e.g., 1:1 to 1:30), further preferably 1:4.75, 1:19 or 1:21.28.

In some embodiments, the method for preparing the pharmaceutical composition of the present invention comprises: co-milling the surface stabilizer with the apixaban to prepare the pharmaceutical composition comprising apixaban nanoparticles; and mixing the sedimentation inhibitor with the preceding pharmaceutical composition, or mixing the sedimentation inhibitor, the remaining portion of the surface stabilizer with the preceding pharmaceutical composition, wherein the surface stabilizer comprises the first surface stabilizer and/or the second surface stabilizer. The preceding method can further comprise a step of lyophilization. For the sake of process and economy, the apixaban and all or portion of the surface stabilizer are generally prepared by co-milling to a concentrated suspension comprising apixaban nanoparticles, which is then diluted to obtain a diluted suspension comprising apixaban nanoparticles suitable for lyophilizing or direct use. The sedimentation inhibitor or the sedimentation inhibitor and the remaining portion of the surface stabilizer may be mixed with the pharmaceutical composition comprising apixaban nanoparticles during dilution, thereby maintaining the stability of the diluted liquid.

The pharmaceutical composition of the present invention may be a lyophilized pharmaceutical composition. In some embodiments, the lyophilized pharmaceutical composition is reconstituted in a liquid medium to obtain a pharmaceutical composition comprising the liquid medium. The liquid medium may be selected from one or more of water, saline solution, safflower seed oil, ethanol, tert-butanol, hexane and ethylene glycol, preferably one or more of water, saline solution and safflower seed oil, most preferably water. In the reconstituted pharmaceutical composition, the content of the apixaban nanoparticle may be selected from 0.1 to 100 mg/mL, preferably 0.5 to 100 mg/mL, more preferably 1 to 100 mg/mL, most preferably 2 to 10 mg/mL (e.g., 2.5 mg/mL).

In some embodiments of the present invention, the pharmaceutical composition comprising the liquid medium after reconstitution can have the technical characteristics of the preceding pharmaceutical composition.

The present invention also provides a pharmaceutical composition comprising a liquid medium, which may be selected from one or more of water, saline solution, safflower seed oil, ethanol, tert-butanol, hexane and ethylene glycol, preferably water, saline solution and safflower seed oil, most preferably water. The content of the apixaban nanoparticle in the pharmaceutical composition comprising the liquid medium may be selected from 0.1 to 100 mg/mL, preferably 0.5 to 100 mg/mL, more preferably 1 to 100 mg/mL, most preferably 2 to 10 mg/mL (e.g., 2.5 mg/mL or 5 mg/mL). When the pharmaceutical composition comprising the liquid medium comprises a sedimentation inhibitor, the content of the apixaban in the pharmaceutical composition may be selected from 0.1 to 100 mg/mL (e.g., 2.5 mg/mL, 5 mg/mL, 50 mg/mL, or 100 mg/mL). When the pharmaceutical composition comprising the liquid medium does not comprise a sedimentation inhibitor, the content of the apixaban in the pharmaceutical composition may be selected from 0.1 to 1000 mg/mL, preferably 1 to 200 mg/mL, more preferably 50 to 100 mg/mL (e.g., 50 mg/mL or 100 mg/mL). The pharmaceutical composition comprising the liquid medium may be lyophilized to prepare the lyophilized pharmaceutical composition.

The pharmaceutical composition of the present invention may be a pharmaceutical composition comprising apixaban nanoparticles and a surface stabilizer, or a nanoparticle suspension comprising apixaban prepared by co-milling apixaban with a surface stabilizer, or the preceding diluted pharmaceutical composition comprising the apixaban nanoparticle suspension, or the lyophilized pharmaceutical composition of the diluted pharmaceutical composition, or the pharmaceutical composition after reconstituting the lyophilized pharmaceutical composition.

The sedimentation inhibitor of the present invention is mixed with the pharmaceutical composition comprising apixaban nanoparticles during dilution. The sedimentation inhibitor can stabilize the diluted suspension comprising apixaban nanoparticles for a period of time. The preferred solution of the present invention is that a portion of the surface stabilizer may be mixed in during dilution, thereby stabilizing the suspension comprising apixaban nanoparticles after dilution. The surface stabilizer is as described above.

The present invention provides an injectable pharmaceutical composition comprising apixaban nanoparticles, a first surface stabilizer, a second surface stabilizer, and a sedimentation inhibitor. The first surface stabilizer, second surface stabilizer, and sedimentation inhibitor are as described above. The weight ratio of the apixaban nanoparticle to the first surface stabilizer may be selected from 1:0.01 to 1:10, preferably 1:0.1 to 1:10, more preferably 1:0.1 to 1:5, further preferably 1:0.1 to 1:3 (e.g., 1:0.2, 1:0.3, 1:1.15 or 1:2.1). The weight ratio of the apixaban nanoparticle to the second surface stabilizer may be selected from 1:0.01 to 1:10, preferably 1:0.1 to 1:5, more preferably 1:0.1 to 1:2, further preferably 1:0.1 to 1:1 (e.g., 1:0.1, 1:0.15, 1:0.575, or 1:1). The weight ratio of the apixaban nanoparticle to the sedimentation inhibitor may be selected from 1:0.1 to 1:100, preferably 1:0.1 to 1:50, more preferably 1:0.5 to 1:30 (e.g., 1:4.75, 1:19 or 1:21.28). The average particle size or D50 of the apixaban nanoparticle may be less than 500 nm, preferably less than 200 nm, further preferably less than 170 nm, further preferably less than 160 nm, further more preferably less than 150 nm.

The present invention provides a method for preparing the pharmaceutical composition, comprising: co-milling a portion of the first surface stabilizer and a portion of the second surface stabilizer with the apixaban to prepare a pharmaceutical composition comprising apixaban nanoparticles; and mixing the sedimentation inhibitor, the other portion of the first surface stabilizer, and the other portion of the second surface stabilizer with the pharmaceutical composition comprising apixaban nanoparticles as described above. The method as described above can further comprise the step of lyophilization.

The present invention provides a pharmaceutical composition comprising apixaban nanoparticle, povidone, and sodium deoxycholate. Further, the weight ratio of the apixaban nanoparticle to the povidone may be selected from 1:0.01 to 1:10, preferably 1:0.1 to 1:10, more preferably 1:0.1 to 1:5, further preferably 1:0.1 to 1:3 or 1:0.1 to 1:2, most preferably 1:0.2 to 1:0.3; the weight ratio of the apixaban nanoparticle to the sodium deoxycholate may be selected from 1:0.01 to 1:10, preferably 1:0.1 to 1:5, more preferably 1:0.1 to 1:1 or 1:0.1 to 1:0.3, most preferably 1:0.1 or 1:0.15.

The present invention provides a pharmaceutical composition comprising apixaban nanoparticle, povidone, sodium deoxycholate, and mannitol. Further, the weight ratio of the apixaban nanoparticle to the povidone may be selected from 1:0.01 to 1:10, preferably 1:0.1 to 1:10, more preferably 1:0.1 to 1:5, further preferably 1:0.1 to 1:3, most preferably 1:1 to 1:3; the weight ratio of the apixaban nanoparticle to the sodium deoxycholate may be selected from 1:0.01 to 1:10, preferably 1:0.1 to 1:5, more preferably 1:0.1 to 1:1, most preferably 1:0.5 to 1:1; the weight ratio of the apixaban nanoparticle to the mannitol may be selected from 1:0.1 to 1:100, preferably 1:0.1 to 1:50, more preferably 1:0.5 to 1:30 (e.g. 1:4.75, 1:21.28).

The present invention provides a pharmaceutical composition comprising apixaban nanoparticle, povidone, sodium deoxycholate, and mannitol, the weight-to-volume ratio of the apixaban nanoparticle in the pharmaceutical composition is preferably 0.1 to 1%, more preferably 0.1 to 0.5% (e.g., 0.25% or 0.5%); the weight-to-volume ratio of the povidone is preferably 0.5 to 5%, more preferably 0.5 to 1 % (e.g., 0.575% or 0.525%); the weight-to-volume ratio of the sodium deoxycholate is preferably 0.1 to 3%, more preferably 0.1 to 0.5% (e.g., 0.2875% or 0.25%); the weight-to-volume ratio of the mannitol is preferably 1 to 10% , more preferably 2 to 7% (e.g., 2.375% or 5.32%).

The present invention provides a pharmaceutical composition, wherein the weight ratio of apixaban nanoparticle: povidone: sodium deoxycholate is 10:2:1, preferably a pharmaceutical composition comprising 10% w/v of apixaban nanoparticle, 2% w/v of povidone and 1% w/v of sodium deoxycholate or the pharmaceutical composition comprising 5% w/v of apixaban nanoparticle, 1% w/v of povidone and 0.5% w/v of sodium deoxycholate.

The present invention further provides a pharmaceutical composition comprising 0.5% w/v of apixaban nanoparticle, 0.575% w/v of povidone, 0.2875% w/v of sodium deoxycholate, and 2.375% w/v of mannitol.

The present invention further provides a pharmaceutical composition comprising 0.25% w/v of apixaban nanoparticle, 0.525% w/v of povidone, 0.25% w/v of sodium deoxycholate, and 5.32% w/v of mannitol.

In some embodiments, in the pharmaceutical composition of the present invention, the content of the apixaban nanoparticle may be 2.0 mg to 5.0 mg per unit dose, preferably 2.0 mg, 2.5 mg, 3.0 mg, 3.5 mg, 4.0 mg, 4.5 mg or 5.0 mg per unit dose. The present invention also provides a method for preparing the pharmaceutical composition comprising co-milling povidone and sodium deoxycholate with apixaban to prepare a pharmaceutical composition comprising apixaban nanoparticles. Further, the pharmaceutical composition comprising apixaban nanoparticles as described above is mixed with mannitol, preferably, the pharmaceutical composition comprising apixaban nanoparticles as described above is diluted with an aqueous solution comprising mannitol. Further, the povidone and sodium deoxycholate are mixed into the pharmaceutical composition in two portions, a portion of the povidone and sodium deoxycholate is co-milled with the apixaban, and the other portion of the povidone and sodium deoxycholate is mixed during dilution, wherein the dilution factor is selected from 1 to 100 times, preferably 5 to 50 times, most preferably 10 to 30 times, and a solvent used for dilution may be selected from the liquid medium as described above. In this solution, "portion" or "the other portion" indicates that the surface stabilizer is mixed into the pharmaceutical composition in two portions.

The present invention provides a pharmaceutical composition prepared by diluting a suspension comprising 10% of apixaban nanoparticle, 2% of povidone, 1% of sodium deoxycholate, and water with a diluent comprising 2.5% of mannitol, 0.5% of povidone, and 0.25% of sodium deoxycholate and a method therefor. The dilution factor may be selected from 1 to 100 times, preferably from 5 to 50 times, most preferably 10 to 30 times, for example may be 10 times, 20 times or 30 times. The method is as follows: diluting the suspension comprising 10% of apixaban nanoparticle, 2% of povidone, 1% of sodium deoxycholate and water with the diluent comprising 2.5% of mannitol, 0.5% of povidone and 0.25% of sodium deoxycholate, wherein the dilution factor may be selected from 1 to 100 times, preferably 5 to 50 times, most preferably 10 to 30 times, for example may be 10 times, 20 times or 30 times.

The present invention provides a pharmaceutical composition prepared by diluting a suspension comprising 5% of apixaban nanoparticle, 1% of povidone, 0.5% of sodium deoxycholate, and water with a diluent comprising 5.6% of mannitol, 0.5% of povidone, and 0.2368% of sodium deoxycholate and a method therefor, wherein a dilution factor may be selected from 1 to 100 times, preferably from 5 to 50 times, most preferably 10 to 30 times, for example may be 10 times, 20 times or 30 times. The method is as follows: diluting the suspension comprising 5% of apixaban nanoparticle, 5% of povidone, 0.5% of sodium deoxycholate and water with the diluent comprising 5.6% of mannitol, 0.5% of povidone and 0.2368% of sodium deoxycholate, wherein the dilution factor may be selected from 1 to 100 times, preferably 5 to 50 times, most preferably 10 to 30 times, for example may be 10 times, 20 times or 30 times.

The present invention provides a pharmaceutical composition prepared by diluting a suspension comprising 10% of apixaban nanoparticle, 2% of povidone, 1% of sodium deoxycholate, and water with a diluent comprising 2.5% of mannitol, 0.5% of povidone, and 0.25% of sodium deoxycholate and a preparation method therefor, wherein the content of the apixaban nanoparticle in the diluted pharmaceutical composition may be selected from 0.1 to 100 mg/mL, preferably 0.5 to 20 mg/mL, more preferably 1 to 10 mg/mL, most preferably 5 to 10 mg/mL. The method is as follows: diluting the suspension comprising 10% of apixaban nanoparticle, 2% of povidone, 1% of sodium deoxycholate and water with the diluent comprising 2.5% of mannitol, 0.5% of povidone and 0.25% of sodium deoxycholate, wherein the content of the apixaban nanoparticle in the diluted pharmaceutical composition may be selected from 0.1 to 100 mg/mL, preferably 0.5 to 20 mg/mL, more preferably 1 to 10 mg/mL, most preferably 5 to 10 mg/mL.

The present invention provides a pharmaceutical composition prepared by diluting a suspension comprising 5% of apixaban nanoparticle, 1% of povidone, 0.5% of sodium deoxycholate, and water with a diluent comprising 5.6% of mannitol, 0.5% of povidone, and 0.2368% of sodium deoxycholate and a preparation method therefor, wherein the content of the apixaban nanoparticle in the diluted pharmaceutical composition may be selected from 0.1 to 100 mg/mL, preferably 0.5 to 20 mg/mL, more preferably 1 to 10 mg/mL, most preferably 1 to 5 mg/mL, such as 1 mg/mL, 1.5 mg/mL, 2 mg/mL, 2.5 mg/mL or 3 mg/mL. The method is as follows: diluting the suspension comprising 5% of apixaban nanoparticle, 5% of povidone, 0.5% of sodium deoxycholate and water with the diluent comprising 5.6% of mannitol, 0.5% of povidone and 0.2368% of sodium deoxycholate, wherein the content of the apixaban nanoparticle in the diluted pharmaceutical composition may be selected from 0.1 to 100 mg/mL, preferably 0.5 to 20 mg/mL, more preferably 1 to 10 mg/mL, most preferably 1 to 5 mg/mL, such as 1 mg/mL, 1.5 mg/mL, 2 mg/mL, 2.5 mg/mL or 3 mg/mL.

The preferred solution of the present invention is to mix the surface stabilizer into the pharmaceutical composition in two portions, a portion of the surface stabilizer is mixed with the apixaban to prepare the pharmaceutical composition comprising apixaban nanoparticles, and the other portion of the surface stabilizer and sedimentation inhibitor is mixed into the pharmaceutical composition comprising apixaban nanoparticles as described above, thereby preparing a more stable diluted pharmaceutical composition comprising apixaban nanoparticles. A solvent in the pharmaceutical composition may be water, which avoids the introduction of organic solvents.

The invention also provides a reconstituted pharmaceutical composition of the lyophilized pharmaceutical composition for injection.

In the pharmaceutical composition comprising apixaban nanoparticles of the present invention, the average particle size of the apixaban nanoparticle may be less than 500 nm, preferably less than 300 nm, more preferably less than 200 nm, further more preferably less than 170 nm, further more preferably less than 160 nm, most preferably less than 150 nm. In the pharmaceutical composition comprising apixaban nanoparticles of the present invention, the PDI of the apixaban nanoparticle may be less than 0.5, preferably less than 0.3, most preferably less than 0.2. In the pharmaceutical composition comprising apixaban nanoparticles of the present invention, the D50 of the apixaban nanoparticle may be less than 500 nm, preferably less than 300 nm, more preferably less than 200 nm, most preferably less than 170 nm.

In the pharmaceutical composition comprising apixaban nanoparticles of the present invention, the average particle size of the apixaban nanoparticle may be less than 500 nm, preferably less than 300 nm, more preferably less than 200 nm, most preferably less than 150 nm, after storage at 2 to 8°C, 25°C/60% RH for a period of time, such as 1 month, 2 months, 3 months, 6 months or 12 months. In the pharmaceutical composition comprising apixaban nanoparticles of the present invention, the PDI of the apixaban nanoparticle may be less than 0.5, preferably less than 0.3, most preferably less than 0.2, after storage at 2 to 8°C, 25°C/60% RH for a period of time, such as 1 month, 2 months, 3 months, 6 months or 12 months. In the pharmaceutical composition comprising apixaban nanoparticles of the present invention, the D50 of the apixaban nanoparticle may be less than 500 nm, preferably less than 300 nm, more preferably less than 200 nm, most preferably less than 170 nm, after storage at 2 to 8°C, 25°C/60% RH for a period of time, such as 1 month, 2 months, 3 months, 6 months or 12 months.

In the pharmaceutical composition comprising apixaban nanoparticles of the present invention, the average particle size of the apixaban nanoparticle may be less than 500 nm, preferably less than 300 nm, more preferably less than 200 nm, further preferably less than 160 nm, after storage at 40°C/75% RH for a period of time, such as 1 month, 2 months, 3 months, 6 months or 12 months. In the pharmaceutical composition comprising apixaban nanoparticles of the present invention, the PDI of the apixaban nanoparticle may be less than 0.5, preferably less than 0.3, most preferably less than 0.2, after storage at 40°C/75% RH for a period of time, such as 1 month, 2 months, 3 months, 6 months or 12 months. In the pharmaceutical composition comprising apixaban nanoparticles of the present invention, the D50 of the apixaban nanoparticle may be less than 500 nm, preferably less than 300 nm, more preferably less than 200 nm, further preferably less than 170 nm, after storage at 40° C/75% RH for a period of time, such as 1 month, 2 months, 3 months, 6 months or 12 months.

In the pharmaceutical composition comprising apixaban nanoparticles of the present invention, the average particle size of the apixaban nanoparticle is basically unchanged after storage at 2 to 8°C, 25°C/60%RH, 40°C/75%RH for a period of time, such as 1 month, 2 months, 3 months, 6 months or 12 months; the PDI of the apixaban nanoparticle is basically unchanged after storage at 2 to 8°C, 25°C/60%RH, 40°C/75%RH for a period of time, such as 1 month, 2 months, 3 months, 6 months or 12 months; the D50 of the apixaban nanoparticle is basically unchanged after storage at 2 to 8°C, 25°C/60%RH, 40°C/75%RH for a period of time, such as 1 month, 2 months, 3 months, 6 months or 12 months. The term "basically unchanged" means that the change is less than 30%, preferably less than 20%, more preferably less than 10%.

Before the storage of the pharmaceutical composition comprising apixaban nanoparticles of the present invention, the content of the apixaban nanoparticle is calculated as 100%; after storage at 2 to 8°C for 5 d, the content of the apixaban nanoparticle relative to the pharmaceutical composition before storage is 95%∼105%, and preferably 98%∼102%; after storage at 25°C/60%RH for 5d, the content of the apixaban nanoparticle relative to the pharmaceutical composition before storage is 95%∼105 %, preferably 98%∼102%; after storage at 40°C/75%RH for 5d, the content of the apixaban nanoparticle relative to the pharmaceutical composition before storage is 95%∼105%, preferably 98% ∼102%. Before the storage of the pharmaceutical composition comprising apixaban nanoparticle of the present invention, the content of the maximum single impurity may be less than 0.5%, preferably less than 0.1 %, most preferably less than 0.05%; after storage at 2 to 8°C for 5d, the content of the maximum single impurity may be less than 0.5%, preferably less than 0.1 %, most preferably less than 0.05%; after storage at 25°C/60%RH for 5d, the content of the maximum single impurity may be less than 0.5%, preferably less than 0.1 %, most preferably less than 0.05%, after storage at 40°C/75%RH for 5d, the content of the maximum single impurity may be less than 0.5%, preferably less than 0.1 %, most preferably less than 0.05%. Before the storage of the pharmaceutical composition comprising apixaban nanoparticles of the present invention, the content of the total impurities may be less than 1%, preferably less than 0.5%; after storage at 2 to 8°C for 5 d, the content of the total impurities may be less than 1%, preferably less than 0.5%; after storage at 25°C/60%RH for 5d, the content of the total impurities may be less than 1%, preferably less than 0.5%; after storage at 40°C/75%RH for 5d, the content of the total impurities may be less than 1%, preferably less than 0.5%.

In the pharmaceutical composition comprising apixaban nanoparticles of the present invention, after storage at 2 to 8°C, 25°C/60%RH, 40°C/75%RH for a period of time, such as 1 month, 2 months, 3 months, 6 months or 12 months, the content of the apixaban nanoparticle relative to the pharmaceutical composition before storage is 95% to 105%, preferably 98% to 102%; the content of the maximum single impurity may be less than 0.5%, preferably less than 0.1 %, most preferably less than 0.05%; the content of the total impurities may be less than 1%, preferably less than 0.5%.

In the pharmaceutical composition comprising apixaban nanoparticles of the present invention, the content of the apixaban nanoparticle is basically unchanged, after storage at 2 to 8°C, 25°C/60%RH, 40°C/75%RH for a period of time, such as 1 month, 2 months, 3 months, 6 months or 12 months; in the pharmaceutical composition comprising apixaban nanoparticles of the present invention, the content of the maximum single impurity is basically unchanged, after storage at 2 to 8°C, 25°C/60%RH, 40°C/75%RH for a period of time, such as 1 month, 2 months, 3 months, 6 months or 12 months; in the pharmaceutical composition comprising apixaban nanoparticles of the present invention, the content of the total impurities is basically unchanged, after storage at 2 to 8°C, 25°C/60%RH, 40°C/75%RH for a period of time, such as 1 month, 2 months, 3 months, 6 months or 12 months. The "basically unchanged" means that the change is less than 20%, preferably less than 10%, more preferably less than 5%.

The pharmaceutical composition comprising apixaban nanoparticles of the present invention is preferably suitable for injection administration, especially for subcutaneous injection administration. The apixaban nanoparticle composition of the present invention have high bioavailability, smooth drug-time profile, and better safety and efficacy after subcutaneous injection. The pharmaceutical composition comprising apixaban nanoparticles of the present invention are preferably suitable for once-daily or twice-daily administration.

The daily dosage of the pharmaceutical composition comprising apixaban nanoparticles of the present invention may be selected from 1 to 10 mg, preferably 2 to 5 mg, more preferably 2 mg, 2.5 mg, 3 mg, 3.5 mg, 4.0 mg, 4.5 mg or 5.0 mg.

A milling device suitable for the present invention comprises dispersion mill such as ball mill, grinder, vibratory mill, and media mill such as sand mill and bead mill. These dispersion mills are well known in the art.

The apixaban of the present invention can represent any factor Xa inhibitor, such as rivaroxaban, edoxaban, betrixaban, etc.

The present invention also relates to a use of the pharmaceutical composition of the present invention in the preparation of drugs for the prevention or treatment of thromboembolic diseases, such as the use of the pharmaceutical compositions comprising apixaban nanoparticles in the preparation of drugs for the prevention or treatment of thromboembolic diseases. Specifically, the present invention also relates to a use of the pharmaceutical composition of the present invention in the preparation of drugs for the prevention or treatment of venous thrombosis, deep vein thrombosis or acute coronary syndrome.

### Detailed description of the invention

In the description and claims of the present application, unless otherwise indicated, the scientific and technical terms used herein have the meanings commonly understood by those skilled in the art. However, in order to better understand the present invention, the definitions and interpretations of some related terms are provided below.

The solvent for diluting the diluent used in the present invention may be a liquid medium; the solvent used for reconstitution may be a liquid medium, and the solvent used for co-milling may be a liquid medium. The liquid medium is the same as described above.

The saline solution described in the present invention includes, but is not limited to, physiological saline solution, buffered saline solution (including, but not limited to, ammonia-ammonium chloride buffer, citrate buffer, acetic acid-sodium acetate buffer and phosphate buffer).

The average particle size described in the present invention refers to the average particle size measured with Malvern nanoparticle size potentiometer Zetasizer Nano ZS.

The content percentage (%) of all substances of the present invention refers to the weight-to-volume ratio, and the "weight-to-volume ratio" of the present invention refers to the weight (unit g) of the component per 100 mL of the liquid system, i.e., g/100 mL. The exceptions are, as in the lyophilized formulation of Embodiment 3: In the four cases of initial stage, 2 to 8°C, 5d, 25°C/60% RH, 5d, 40°C/75% RH, 5d. 25°C/60% RH, 5d, 40°C/75% RH, 5d, the apixaban content/% refers to the content of apixaban under other storage conditions measured using HPLC method with starting content as 100%; the content of the maximum single impurity and total impurities were obtained by the HPLC method and the peak area ratio was calculated. When the substances in the pharmaceutical composition of the present invention are expressed by weight-to-volume ratio, the pharmaceutical composition further comprises a liquid medium, and the liquid medium is the same as described above.

The "PDI" in the present invention refers to the polydispersity index of the particle size distribution width of a sample. The "D50" refers to the particle size of a sample when the light energy is accumulated to 50%, and the definition of D10 and D90 is analogous to D50.

The content (including percentage content) of various substances and the ratio between these substances described in the present invention are all allowable to have an error of ±5%, e.g., "the content of apixaban nanoparticle in the composition is 5 to 10 mg/mL" means that a 4.75 to 10.5 mg/mL of the apixaban nanoparticle in the composition is within the scope of the present invention; "comprising 10% of apixaban nanoparticle" means that comprising 9.5 to 10.5% of apixaban nanoparticle is within the scope of the present invention; "the weight ratio of the apixaban nanoparticle to the sedimentation inhibitor is selected from 1:0.1 to 1:100" means that the weight ratio of the apixaban nanoparticle to the sedimentation inhibitor selecting from 1:0.095 to 1:105 is within the scope of the present invention. In the present invention, "about" means a technical solution comprising an error of ±5%.

The "mix into" and "mix with" in the present invention mean that the order of addition of the components is not limited. For example, if A is mixed into B, it may mean that A is added to B, or it also may mean that B is added to A. If A is mixed with B, it may mean that A is mixed with B, or it also may mean that B is mixed with A.

### Beneficial effects of the invention

The present invention improves the dispersibility of nanoparticle in a suspension by adding a surface stabilizer to a pharmaceutical composition comprising apixaban nanoparticles for injection. The present invention inhibits the sedimentation or aggregation of nanoparticles by adding a sedimentation inhibitor such as mannitol to the composition. In the preferred technical solution of the present invention, a portion of surface stabilizer is added in the process of diluting the suspension, which further improves the stability of the diluted solution of the suspension. The present invention removes the liquid medium such as water used in the preparation process by lyophilization, as well as prevents the generation of hydrolytic impurities, thereby improving the stability of the nanoparticle in the pharmaceutical composition during long-term storage and promoting their clinical application.

### Detailed description of the preferred embodiment

The present invention is further described in detail by the following embodiments and experimental embodiments. These embodiments and experimental embodiments are for illustrative purposes only and are not intended to limit the scope of the present invention.

The method for detecting the particle size of nanoparticle injections in the following examples refers to the third method of the Chinese Pharmacopoeia 2015 Edition Four General Rules 0982.

Malvern nanoparticle size potentiometer Zetasizer Nano ZS is used for measuring particle size, and the following parameters are adopted, i.e., dispersion medium: purified water; dilution factor: diluted to the concentration of the apixaban about 0.1mg/ml; test temperature: 25°C; absorption rate: 0.01; refractive index 1.59; equilibrium time: 120s; test location: optimal position.

In the following embodiments, the impurity content in the nanoparticle injection was detected by HPLC, and the detection conditions are as follows: octadecylsilane-bonded silica gel is used as filler (Waters Xbridge Shield RP₁₈, 3.5 µm, 4.6×150 mm), the mobile phase is acetonitrile /ammonium acetate buffer solution, and the detection wavelength is 280 nm.

### Comparative embodiment 1 Prescription of apixaban nanoemulsion formulation

**Table 1 Prescription of apixaban nanoemulsion formulation**

| Nanoemulsion | | | |
|---|---|---|---|
| Material name | Prescribed amount (g) | Concentration (mg/mL) | Function |
| Apixaban | 0.2 | 2 | Principal component |
| Polyethylene glycol 15-hydroxy stearate | 4.4 | 44 | Emulsifier |
| Medium chain triglyceride | 1.1 | 11 | Solubilizer |
| Soybean oil | 0.66 | 6.6 | Solubilizer |
| Sodium chloride | 0.623 | 6.23 | Osmoregulator |
| Anhydrous disodium hydrogen phosphate | 0.284 | 2.84 | Buffer |
| Hydrochloric acid or sodium hydroxide | Appropriate amount | Appropriate amount | pH regulator |
| Water for injection | Add to 100 mL | Add to full volume | Solvent |

Preparation method is as follows: A prescribed amount of polyethylene glycol 15-hydroxystearate, medium chain triglyceride and soybean oil were mixed, and heated to 60±5°C. About 20% of the total amount of water for injection with a temperature of 45±5°C was added to the oil phase, and stirred at 300 rpm for not less than 20 min to prepare colostrum. A prescribed amount of apixaban raw material was added to colostrum, and the mixture was stirred at 45±5°C, 300rpm for not less than 30 minutes until the raw materials were dissolved. Water for injection at 15 to 30°C was used to dilute the emulsion to 90% of the total amount and stirring evenly. A mixed aqueous solution of anhydrous disodium hydrogen phosphate and sodium chloride was added, and the mixture was stirred evenly, thereby addjusting the pH to a range of 7.0 to 8.0. Finally, the water for injection was added to full volume, and at this moment, the precipitation of API was observed.

### Comparative embodiment 2 Prescription of cyclodextrin inclusion compound formulation

**Table 2 Prescription of cyclodextrin inclusion compound formulation**

| Cyclodextrin inclusion compound | | | |
|---|---|---|---|
| Material name | Prescribed amount (g) | Concentration (mg/mL) | Function |
| Apixaban | 0.25 | 2.5 | Principal component |
| Hydroxypropyl-β-cyclodextrin | 20 | 200 | Solubilizer |
| PEG400 | 25 | 250 | Solubilizer |
| Water for injection | Add to 100 mL | Add to full volume | Solvent |

Preparation method is as follows: A prescribed amount of hydroxypropyl-β-cyclodextrin and PEG400 were dissolved with appropriate amount of water, and apixaban raw material was added. Water was made up to the full volume, and the mixture was stirred to dissolve, and at this moment, the precipitation of API had been observed.

### Embodiment 1 Prescription of apixaban nanoparticle suspensions

**Table 3 Prescription of apixaban nanoparticle suspensions**

| Prescription | Average particle size /nm | PDI | D50/nm |
|---|---|---|---|
| Prescription (1) : 10% of apixaban+2% of poloxamer+1% of sodium deoxycholate | 283.3 | 0.231 | 286 |
| Prescription (2) : 10% of apixaban+2% of poloxamer | 1372 | 0.536 | 809 |
| Prescription (3) : 10% of apixaban+3% | 175.6 | 0.241 | 200 |
| of povidone+1.5% of sodium deoxycholate | | | |
| Prescription (4) : 10% of apixaban+2% of povidone+1% of sodium deoxycholate | 140.3 | 0.203 | 151 |
| Prescription (5) : 5% of apixaban+1% of povidone+0.5% of sodium deoxycholate | 132.7 | 0.156 | 146 |
| Prescription (6) : 10% of apixaban+2% of povidone+1% of docusate sodium | 254.9 | 0.208 | 262 |
| Prescription (7) : 10% of apixaban+2% of Tween 80 | 269.7 | 0.279 | 282 |

Preparation method is as follows: A prescribed amount of surface stabilizer was dissolved with water, apixaban raw material was added, and the mixture was stirred to make the dispersion uniform. Grinding beads were added and then the mixture was stirred to make the dispersion uniform, and the mixture was milled on a ball mill for 4h to prepare an apixaban nanoparticle suspension.

Comparing the drug loading of the formulations of comparative embodiment 1 and 2 with that of embodiment 1, it was found that apixaban cannot be effectively solubilized in the formulations of Comparative Embodiment 1 and 2, and the drug loading of apixaban in the prescription of Embodiment 1 can reach 100 mg/mL.

Except for prescription (2), the average particle size of the apixaban nanoparticle suspension in the present invention is between 100 to 500 nm, the PDI is below 0.5, and the particle size and distribution of the drug are ideal.

### Embodiment 2 Dilution and lyophilization of apixaban nanoparticle suspensions of prescription (4) and (5)

**Table 4 Dilution and lyophilization of apixaban nanoparticle suspensions**

| Prescription (4) | | | | |
|---|---|---|---|---|
| Diluent | Storage condition | Average particle size /nm | PDI | D50/n m |
| - | After milling | 128.0 | 0.198 | 140 |
| 2.5% of mannitol | Before lyophilization | 134.8 | 0.229 | 139 |
| 10% of sucrose | Before lyophilization | 139.8 | 0.226 | 145 |
| 2.5% of mannitol+0.5% of povidone+0.25% of sodium deoxycholate | Before lyophilization | 133.9 | 0.215 | 145 |
| 2.5% of mannitol | 2 to 8°C, 4d | 200.3 | 0.234 | 201 |
| 10% of sucrose | 2 to 8°C, 4d | 216.4 | 0.242 | 219 |
| 2.5% of mannitol+0.5% of povidone+0.25% of sodium deoxycholate | 2 to 8°C, 4d | 145.8 | 0.262 | 153 |
| 2.5% of mannitol | After lyophilization | 149.1 | 0.255 | 152 |
| 10% of sucrose | After lyophilization | 154.7 | 0.215 | 164 |
| 2.5% of mannitol+0.5% of povidone+0.25% of sodium deoxycholate | After lyophilization | 138.8 | 0.226 | 150 |

| Prescription (5) | | | | |
|---|---|---|---|---|
| 5.6% of mannitol+0.5% of povidone+0.2368% of sodium deoxycholate | After milling | 132.7 | 0.156 | 146 |
| | Before lyophilization | 122.1 | 0.101 | 130 |
| | 2 to 8°C, 24h | 126.3 | 0.130 | 137 |
| | After lyophilization | 126.0 | 0.131 | 136 |

Preparation method is as follows: The apixaban nanoparticle suspension of prescription (4) prepared in Embodiment 1 was diluted with 2.5% of mannitol, 2.5% of glycerol aqueous solution, 10% of sucrose aqueous solution, 2.5% of mannitol + 0.5% of povidone + 0.25% of sodium deoxycholate aqueous solution until the concentration of apixaban is 5 mg/mL, respectively, and the particle size and distribution of nanoparticle in the three dilutions were measured respectively; the diluent was stored at 2 to 8°C for 4 days, and the particle size and distribution of nanoparticle in the three dilutions were measured respectively; or the diluent was directly lyophilized, and the particle size and distribution of nanoparticle after the three diluents were lyophilized in the three diluents were measured respectively. The apixaban nanoparticle suspension of prescription (5) prepared in Embodiment 1 was diluted with 5.6% of mannitol + 0.5% of povidone + 0.2368% of sodium deoxycholate aqueous solution until the concentration of apixaban is 2.5 mg/mL, and the particle size and distribution of nanoparticle were measured respectively after milling, before lyophilization, stored at 2 to 8°C for 24 h, after lyophilization (Note: the nanoparticle suspension of prescription (5) was diluted, sterilized through a 0.22 micron filter element, and then lyophilized).

### Embodiment 3 Stability investigation of lyophilized formulations of prescriptions (4) and (5)

The apixaban nanoparticle suspension of prescription (4) was diluted with an aqueous solution comprising 2.5% of mannitol + 0.5% of povidone + 0.25% of sodium deoxycholate, and then the lyophilized formulation was placed under the conditions of 2 to 8°C, 25°C/60% RH and 40°C/75% RH, respectively, and after reconstituting with water, the particle size distribution, content and related substances were measured, and the stability was monitered, see Table 5 (batch 1) for details. The apixaban nanoparticle suspension of prescription (5) was diluted with an aqueous solution comprising 5.6% of mannitol + 0.5% of povidone + 0.2368% of sodium deoxycholate, and then the lyophilized formulation was placed under the conditions of 25°C/60%RH and 40°C/75% respectively, and after reconstituting with water, the particle size distribution, content and related substances were measured, and the stability was monitered, see Table 5 (batch 2, 3, 4) for details. The apixaban nanoparticle suspension of prescription (5) was diluted with an aqueous solution comprising 5.6% of mannitol + 0.5% of povidone + 0.2368% of sodium deoxycholate, then lyophilized, and reconstituted with water. The particle size distributions were investigated at room temperature and 2 to 8°C after 1h, 2h, 4h, 6h, 24h, 48h, see Table 6 for details.

**Table 5 Stability investigation of lyophilized formulations of prescriptions (4) and (5)**

| Batch | Storage condition | Average particle size /nm | PDI | D50/n m | Content/ % | Maximum single impurity /% | Total impurities /% |
|---|---|---|---|---|---|---|---|
| 1 | Initial stage | 138.8 | 0.226 | 150 | 100.00 | 0.02 | 0.20 |
| | 2 to 8°C, 5d | 136.9 | 0.227 | 146 | 101.22 | 0.02 | 0.20 |
| | 25°C/60% RH, 5d | 137.8 | 0.214 | 147 | 101.11 | 0.02 | 0.19 |
| | 40°C/75% RH, 5d | 137.9 | 0.225 | 148 | 100.66 | 0.02 | 0.19 |
| | 2 to 8°C, 1M | 136.5 | 0.215 | 147 | 99.89 | 0.02 | 0.20 |
| | 25°C/60% RH, 1M | 135.6 | 0.215 | 148 | 100.00 | 0.02 | 0.21 |
| | 40°C/75% RH, 1M | 139.2 | 0.223 | 149 | 100.88 | 0.02 | 0.21 |
| 2 | Initial stage | 143 | 0.171 | 156 | 120.6 | 0.24 | 0.86 |
| | 25°C/60% RH, 1M | 141.6 | 0.163 | 155 | 119.2 | 0.21 | 0.66 |
| | 25°C/60% RH, 2M | 144.9 | 0.159 | 158 | 119.9 | 0.21 | 0.70 |
| | 25°C/60% RH, 3M | 147.5 | 0.168 | 158 | 122.1 | 0.22 | 0.84 |
| | 40°C/75% RH, 1M | 146.9 | 0.157 | 161 | 119.4 | 0.23 | 0.82 |
| | 40°C/75% RH, 2M | 147.3 | 0.160 | 161 | 119.4 | 0.22 | 0.73 |
| | 40°C/75% RH, 3M | 151.2 | 0.167 | 168 | 121.9 | 0.23 | 0.88 |
| 3 | Initial stage | 142.4 | 0.168 | 156 | 119.0 | 0.24 | 0.87 |
| | 25°C/60% RH, 1M | 144.1 | 0.171 | 155 | 115.0 | 0.20 | 0.62 |
| | 25°C/60% RH, 2M | 146 | 0.166 | 156 | 119.7 | 0.21 | 0.71 |
| | 25°C/60% RH, 3M | 148.9 | 0.176 | 158 | 121.6 | 0.22 | 0.87 |
| | 40°C/75% RH, 1M | 150.6 | 0.172 | 163 | 118.6 | 0.19 | 0.67 |
| | 40°C/75% RH, 2M | 152 | 0.166 | 163 | 120.4 | 0.21 | 0.72 |
| | 40°C/75% RH, 3M | 158.6 | 0.185 | 166 | 121.6 | 0.22 | 0.83 |
| 4 | Initial stage | 141.8 | 0.162 | 156 | 121.3 | 0.21 | 0.76 |
| | 25°C/60% RH, 1M | 142.4 | 0.173 | 155 | 120.4 | 0.16 | 0.63 |
| | 25°C/60% RH, 2M | 144.2 | 0.172 | 160 | 120.4 | 0.21 | 0.72 |
| | 25°C/60% RH, 3M | 147 | 0.160 | 161 | 122.1 | 0.23 | 0.87 |
| | 40°C/75% RH, 1M | 146.3 | 0.154 | 159 | 120.4 | 0.19 | 0.67 |
| | 40°C/75% RH, 2M | 151.9 | 0.159 | 166 | 121.0 | 0.21 | 0.72 |
| | 40°C/75% RH, 3M | 155.1 | 0.167 | 163 | 122.2 | 0.23 | 0.86 |

**Table 6 Stability of particle size distribution**

| Conditions for sample retention | Average particle size /nm | PDI | D(10) /nm | D(50) /nm | D(90)/ nm |
|---|---|---|---|---|---|
| 0h | 127.5 | 0.131 | 83.2 | 137 | 228 |
| Room temperature, 1h | 128.1 | 0.138 | 81.7 | 138 | 238 |
| Room temperature, 2h | 127.3 | 0.121 | 83.8 | 137 | 224 |
| Room temperature, 4h | 128 | 0.152 | 80.7 | 140 | 244 |
| Room temperature, 6h | 129.2 | 0.123 | 86.7 | 140 | 225 |
| Room temperature, 24h | 132.6 | 0.141 | 86.9 | 145 | 243 |
| Room temperature, 48h | 131.9 | 0.143 | 83.2 | 143 | 248 |

It can be seen from the results in Table 5 that the lyophilized formulations of apixaban nanoparticle are relatively stable under different conditions, and the particle size distribution, content and related substances do not change significantly compared with the starting ones. It can be seen from the results in Table 6 that the particle size distribution of apixaban nanoparticle lyophilized formulations is uniform after reconstitution, indicating that the stability of the particle size distribution of lyophilized formulations of apixaban nanoparticle is good after reconstitution. The content of apixaban and related substances were determined by HPLC.

### Embodiment 4 Pharmacokinetics study

The apixaban nanoparticle suspension of prescription (5) was diluted with an aqueous solution comprising 5.6% of mannitol + 0.5% of povidone + 0.2368% of sodium deoxycholate, lyophilized, and reconstituted with water to prepare 2.5 mg/mL apixaban injection. 1 mL of apixaban injection was administered subcutaneously to three subjects respectively, and the plasma drug concentrations are shown in Table 7.

**Table 7 Changes of plasma drug concentration (ng/mL) of apixaban over time**

| Time (h) | C-01 | C-02 | C-03 | Mean | SD |
|---|---|---|---|---|---|
| 0 | 0 | 0 | 0 | 0 | NA |
| 0.25 | 8.08 | 5.36 | 5.13 | 6.19 | 1.6 |
| 0.5 | 18.2 | 14.3 | 10.9 | 14.5 | 3.6 |
| 1 | 35.8 | 23.8 | 21.7 | 27.1 | 7.6 |
| 2 | 52.0 | 37.9 | 31.9 | 40.6 | 10.3 |
| 3 | 62.1 | 43.2 | 36.6 | 47.3 | 13.2 |
| 4 | 68.8 | 44.8 | 40.0 | 51.2 | 15.4 |
| 5 | 52.4 | 38.1 | 39.7 | 43.4 | 7.8 |
| 6 | 48.8 | 40.4 | 35.5 | 41.6 | 6.8 |
| 8 | 46.7 | 33.4 | 37.6 | 39.3 | 6.8 |
| 10 | 37.6 | 28.9 | 30.1 | 32.2 | 4.7 |
| 16 | 22.8 | 19.2 | 21.6 | 21.2 | 1.8 |
| 24 | 14.3 | 11.4 | 15.8 | 13.8 | 2.3 |

The experimental data showed that the half-life of the subcutaneous injection of the apixaban nanoparticle pharmaceutical composition is 11.1 hours, indicating that subcutaneous administration of nanoparticle pharmaceutical composition has a sustained release effect in humans; the PK curve is relatively smooth, and the drug concentration in blood is higher than 21 ng/mL from 1 to 16 hours after administration, which can meet the way of administering once a day; the Cₘₐₓ of a single subcutaneous administration of 2.5 mg is low (51.2), and the safety of administration is controllable.

Although specific embodiments of the present invention have been described above, those skilled in the art will understand that these are merely illustrative, and various changes or modifications may be made to these embodiments without departing from the principles and substances of the present invention. Accordingly, the scope of protection of the present invention is defined by the appended claims.

## Claims

1. An injectable pharmaceutical composition, comprising apixaban nanoparticles and a surface stabilizer.

2. The pharmaceutical composition as defined in claim 1, wherein, the surface stabilizer is selected from one or more of nonionic surface stabilizers, anionic surface stabilizers, cationic surface stabilizers and zwitterionic surface stabilizers, preferably one or more of povidone, polyvinyl alcohol, docusate sodium, hydroxypropyl methylcellulose, Tween 80, lecithin, sodium deoxycholate, sodium cholate, poloxamer, and polyethylene glycol 15-hydroxystearate.

3. The pharmaceutical composition as defined in claim 2, wherein the apixaban nanoparticle and the surface stabilizer are present in a weight ratio selected from 1:0.01 to 1:100, preferably 1:0.1 to 1:10, more preferably 1:0.2 to 1:5, most preferably 1:0.3 to 1:4.

4. The pharmaceutical composition as defined in claim 1, wherein, the surface stabilizer comprises a first surface stabilizer and/or a second surface stabilizer.

5. The pharmaceutical composition as defined in claim 4, wherein, the first surface stabilizer is selected from nonionic surface stabilizers and/or zwitterionic surface stabilizers, preferably one or more of povidone, polyvinyl alcohol, hydroxypropyl methylcellulose, Tween 80, poloxamer, polyethylene glycol 15-hydroxystearate and lecithin, more preferably one or more of povidone, poloxamer and Tween 80.

6. The pharmaceutical composition as defined in claim 5, wherein, the second surface stabilizer is selected from anionic surface stabilizers, preferably one or more of sodium deoxycholate, sodium cholate and docusate sodium, more preferably sodium deoxycholate.

7. The pharmaceutical composition as defined in any one of claims 4-6, wherein, the apixaban nanoparticles and the first surface stabilizer are present in a weight ratio selected from 1:0.01 to 1:10, preferably 1:0.1 to 1:10, more preferably 1:0.1 to 1:5, most preferably 1:0.1 to 1:3.

8. The pharmaceutical composition as defined in claim 7, wherein, the apixaban nanoparticles and the second surface stabilizer are present in a weight ratio selected from 1:0.01 to 1:10, preferably 1:0.1 to 1:5, more preferably 1:0.1 to 1:2.

9. The pharmaceutical composition as defined in any one of claims 1-8, wherein, the apixaban nanoparticle has an average particle size or D50 of less than 500 nm, preferably less than 200 nm, further preferably less than 170 nm.

10. The pharmaceutical composition as defined in any one of claims 1-9, wherein, the apixaban is co-milled with a portion of or all the surface stabilizer to obtain the pharmaceutical composition comprising apixaban nanoparticles; wherein, when the apixaban is co-milled with a portion of the surface stabilizer, the apixaban and the portion of the surface stabilizer are preferably present in a weight ratio selected from 1:2 to 1:100, preferably 1:2 to 1:50, more preferably 1:2 to 1:20, most preferably 1:3 to 1:15.

11. The pharmaceutical composition as defined in any one of claims 1-10, wherein, the pharmaceutical composition further comprises a sedimentation inhibitor.

12. The pharmaceutical composition as defined in claim 11, wherein, the sedimentation inhibitor is selected from one or more of mannitol, sucrose, dextran 40, trehalose, glycerol, povidone, glycine, and hydroxypropyl-β-cyclodextrin, preferably one or more of mannitol, sucrose and dextrose 40, more preferably mannitol.

13. The pharmaceutical composition as defined in claim 12, wherein, the apixaban nanoparticles and the sedimentation inhibitor are present in a weight ratio selected from 1:0.1 to 1:100, preferably 1:0.1 to 1:50, more preferably 1:0.5 to 1:30, most preferably 1:1 to 1:30.

14. The pharmaceutical composition as defined in any one of claims 1-13, wherein, the pharmaceutical composition is a lyophilized pharmaceutical composition.

15. The pharmaceutical composition as defined in any one of claims 1-13, wherein, the pharmaceutical composition comprises a liquid medium which is preferably selected from one or more of water, saline solution and safflower seed oil, more preferably water.

16. The pharmaceutical composition as defined in claim 15, wherein, the apixaban nanoparticles have a content selected from 0.1 to 100 mg/mL, preferably 0.5 to 100 mg/mL, more preferably 1 to 100 mg/mL, most preferably 2 to 10 mg/mL.

17. A lyophilized pharmaceutical composition, which is obtained by lyophilizing the pharmaceutical composition as defined in claim 15 or 16, or the lyophilized pharmaceutical composition is reconstituted in a liquid medium to obtain the composition as defined in claim 15 or 16, wherein the liquid medium used for reconstituting is preferably water.

18. An injectable pharmaceutical composition, comprising apixaban nanoparticles, a first surface stabilizer, a second surface stabilizer, and a sedimentation inhibitor; wherein, the first surface stabilizer is preferably selected from nonionic surface stabilizers and/or zwitterionic surface stabilizers, further preferably one or more of povidone, polyvinyl alcohol, hydroxypropyl methylcellulose, Tween 80, poloxamer, polyethylene glycol 15-hydroxystearate and lecithin, more preferably one or more of povidone, poloxamer and Tween 80; the second surface stabilizer is preferably selected from anionic surface stabilizers, further preferably one or more of sodium deoxycholate, sodium cholate and docusate sodium, more preferably sodium deoxycholate; the sedimentation inhibitor is preferably selected from one or more of mannitol, sucrose, dextran 40, trehalose, glycerol, povidone, glycine, and hydroxypropyl-β-cyclodextrin, further preferably one or more of mannitol, sucrose and dextrose 40, more preferably mannitol; the apixaban nanoparticle preferably has an average particle size or D50 of less than 500 nm, preferably less than 200 nm.

19. A pharmaceutical composition, comprising apixaban nanoparticles, povidone, sodium deoxycholate, and mannitol, the apixaban nanoparticle and the povidone are preferably present in a weight ratio selected from 1:0.01 to 1:10, the apixaban nanoparticle and the sodium deoxycholate are preferably present in a weight ratio selected from 1:0.01 to 1:10; and the apixaban nanoparticle and the mannitol are preferably present in a weight ratio selected from 1:0.1 to 1:100.

20. A pharmaceutical composition, comprising apixaban nanoparticles, povidone, sodium deoxycholate and mannitol, wherein the apixaban nanoparticles in the pharmaceutical composition are preferably 0.1 to 1% w/v, povidone is preferably 0.5 to 5% w/v and sodium deoxycholate is preferably 0.1 to 3% w/v.

21. The pharmaceutical composition as defined in any one of claims 1-20, wherein, in a unit dose the content of apixaban nanoparticles is 2.5mg.

22. A pharmaceutical composition, comprising apixaban nanoparticle, povidone and sodium deoxycholate in a weight ratio of 10:2:1, preferably (i) comprising 10% w/v of apixaban nanoparticle, 2% w/v of povidone and 1% w/v of sodium deoxycholate, or (i i) comprising 5% w/v of apixaban nanoparticle, 1% w/v of povidone and 0.5% w/v of sodium deoxycholate.

23. The pharmaceutical composition as defined in claim 20, wherein, the pharmaceutical composition comprises (i) 0.5% w/v of apixaban nanoparticle, 0.575% w/v of povidone, 0.2875% w/v of sodium deoxycholate and 2.375% of mannitol, or (ii) 0.25% w/v of apixaban nanoparticle, 0.525% w/v of povidone, 0.25% w/v of sodium deoxycholate and 5.32% w/v of mannitol.

24. A method for preparing the pharmaceutical composition as defined in any one of claims 1-22, comprising co-milling the surface stabilizer and the apixaban to prepare the pharmaceutical composition comprising apixaban nanoparticles.

25. A method for preparing the pharmaceutical composition as defined in any one of claims 4-10, comprising co-milling the first surface stabilizer and/or the second surface stabilizer with the apixaban to prepare the pharmaceutical composition comprising apixaban nanoparticles.

26. A method for preparing the pharmaceutical composition as defined in any one of claims 11-18, comprising co-milling the surface stabilizer with the apixaban to prepare the pharmaceutical composition comprising apixaban nanoparticles, and mixing the sedimentation inhibitor with the preceding pharmaceutical composition, wherein the surface stabilizer preferably comprises the first surface stabilizer and/or the second surface stabilizer.

27. A method for preparing the pharmaceutical composition as defined in any one of claims 11-18, comprising co-milling a portion of the first surface stabilizer and a portion of the second surface stabilizer with the apixaban to prepare a pharmaceutical composition comprising apixaban nanoparticles; and mixing the sedimentation inhibitor, the other portion of the first surface stabilizer, and the other portion of the second surface stabilizer with the preceding pharmaceutical composition comprising apixaban nanoparticles.

28. The method as defined in any one of claims 24-27, wherein, the method further comprises a step of lyophilization.

29. A method for preparing the pharmaceutical composition as defined in any one of claims 19-23, comprising co-milling povidone and sodium deoxycholate with apixaban to prepare the pharmaceutical composition comprising apixaban nanoparticles.

30. A method for preparing the pharmaceutical composition as defined in any one of claims 19-23, comprising co-milling povidone and sodium deoxycholate with apixaban to prepare the pharmaceutical composition comprising apixaban nanoparticles, and mixing mannitol with the said pharmaceutical composition comprising apixaban nanoparticles.

31. The method as defined in claim 30, wherein, the povidone and sodium deoxycholate are mixed into the pharmaceutical composition in two portions, a portion of the povidone and sodium deoxycholate is co-milled with the apixaban, and the other portion of the povidone and sodium deoxycholate is mixed during dilution, wherein the dilution factor preferably selected from 1 to 100 times, preferably 5 to 50 times, most preferably 10 to 30 times.

32. A method for preparing the pharmaceutical composition as defined in claim 23, comprising (i) diluting a suspension comprising 10% of apixaban nanoparticles, 2% of povidone, 1% of sodium deoxycholate, and water with a diluent comprising 2.5% of mannitol, 0.5% of povidone, and 0.25% of sodium deoxycholate, wherein in the diluted pharmaceutical composition the content of apixaban nanoparticles is preferably 0.1 to 100 mg/mL, preferably 0.5 to 20 mg/mL, more preferably 1 to 10 mg/mL, most preferably 5 to 10 mg/mL; or (ii) diluting a suspension comprising 5% of apixaban nanoparticle, 1% of povidone, 0.5% of sodium deoxycholate, and water with a diluent comprising 5.6% of mannitol, 0.5% of povidone, and 0.2368% of sodium deoxycholate, wherein in the diluted pharmaceutical composition the content of apixaban nanoparticles is preferably 0.1 to 100 mg/mL, preferably 0.5 to 20 mg/mL, more preferably 1 to 10 mg/mL, most preferably 1 to 5 mg/mL.

33. The method as defined in any one of claims 30-32, wherein the method may further comprise a step of lyophilizing the diluted pharmaceutical composition.

34. A use of the pharmaceutical composition as defined in any one of claims 1-23 in the preparation of medicants for the prevention or treatment of venous thrombosis.

35. The pharmaceutical composition as defined in any one of claims 1-23, wherein the pharmaceutical composition is suitable for administration by subcutaneous injection.

36. The pharmaceutical composition as defined in any one of claims 1-23, wherein the pharmaceutical composition is suitable for once-daily administration.
